# EUROPEAN PATENT APPLICATION

(11) **EP 1 584 677 A1**
(43) Date of publication of application: **12.10.2005**
(21) Application number: 04090136.5
(22) Date of filing: 07.04.2004
(51) Int. Cl.: C12N 9/22

(54) **EcoP151 - process conditions and an efficient method for its large-scale purification**

(71) Applicant: Charité - Universitätsmedizin Berlin, 10117 Berlin (DE)
(72) Inventor: Krüger, Detlev H., Prof. Dr., 14532 Stahnsdorf (DE); Möncke-Buchner, Elisabeth, 14197 Berlin (DE); Mackeldanz, Petra, 10317 Berlin (DE); Reuter, Monika, 13156 Berlin (DE)
(74) Representative: Rasch, Dorit

(57) **Abstract**

The present invention relates to a recombinant DNA which encodes a storable EcoP15I type III restriction endonuclease as well as large-scale purification of EcoP15I to near homogeneity and the use of said EcoP15I as tool for serial analysis of gene expression and/or the identification of corresponding genes.

## Description

The present invention relates to a recombinant DNA which encodes an EcoP15I type III restriction endonuclease with a non-limited storage stability as well as large-scale purification of EcoP15I to near homogeneity and the use of said EcoP15I as tool for serial analysis of gene expression and/or the identification of corresponding genes.

A wide variety of methods have recently been developed to detect and characterize gene expression and/or the identification of corresponding genes. Modification of the structure and expression of genes by genetic engineering provides enormous potential for various medical, pharmaceutical and agricultural applications.

In order to detect and select target genes for engineering, screening of cells, tissues, organs or organisms in different developmental stages and under a variety of environmental conditions (e.g. stress) expression profiling methods are massively applied. One of the most powerful techniques for gene expression analysis is SAGE (Serial Analysis of Gene Expression): Briefly, complementary DNA (cDNA) is reverse-transcribed from mRNA isolated from defined cells, tissues or organs by reverse transcriptase using a biotinylated oligo-dT primer. Generated single-stranded cDNA is converted into double-stranded DNA, and digested with restriction enzyme NlaIII, that recognizes the sequence motif 5'-CATG-3'. The 3'-end fragments of the double-stranded cDNA are recovered by streptavidin-coated magnetic beads, and divided into two portions. Two different linkers are then ligated to each of the cDNA portions. The linkers contain the sequence motif 5'-GGGAC-3', that is the recognition site of the type II restriction enzyme BsmFI, which cleaves 13-15 bp apart from the recognition site in the 3'-direction. Thus, treatment of the linkered cDNAs with *BsmF* releases a 13bp fragment of the cDNA, called "tag" sequence, together with the linker fragment ("linker-tag" fragment). After blunting the staggered ends of tag fragments, two of the "linker-tag" fragments generated from two portions of cDNAs are ligated to each other such that two randomly chosen tags are in adjacent position to form a "ditag", which is subsequently amplified by PCR with primers specific to the linker sequence. After removing the linker fragment by NlaIII digestion, the "ditags" are concatenated, and cloned into an appropriate plasmid. Sequencing of the plasmid insert shows a series of 9bp tags flanked by the invariable 4 bp 5'-CATG-3' *Nla*III recognition sequence. Using the 13 bp tag sequence, in many cases it is possible to identify the gene from which the tag sequence originated, by consulting available expressed sequence tag (EST) databases. Thus, after sequencing thousands of tags, it is possible to count the number of each tag of transcripts in the sample, and further to describe the gene expression profile in the sample. The SAGE protocol described above is therefore an effective quantitative method to study global gene expression. However, the limited size of the tag sequence, only 13 bp, is not always sufficient to unequivocally identify the gene from which the tag derived. A single tag sequence may correspond to several different EST and genomic sequences, and may confound further analysis.

To improve the situation, a so-called "SuperSAGE" protocol was developed that allows the isolation of tag fragment larger than 25-bp in size.

The use of a type III restriction enzyme, EcoP15I, as a tagging enzyme is the key feature of SuperSAGE that enabled the isolation of 26-bp tags. Among all the functionally analysed restriction enzymes, EcoP15I has the cleavage site most distant from the recognition site. Restriction enzymes of type III are enzymes with extremely high specificity which recognize and cleave a particular base sequence in deoxyribonucleic acid (DNA); with this excellent specificity, they are widely used in the field of genetic engineering.

Regrettably, there are no current methods available for large-scale purification of complete a type III restriction enzyme, particularly EcoP15I. For instance the so-called purified EcoP15I of prior art is a mixture (i) of DNA methyltransferase (Mod₂) and (ii) restriction endonuclease (Mod₂Res₂). Unfortunately, (a) for 1 mg purified enzyme 7 - 8 1 bacterial culture solution are necessary and (b) the whole process of purification takes 10 - 14 days, whereby (c) yield of the desired product is 0,1 mg EcoP15I per 1 g cells. An additional problem is the limited storage stability of known EcoP15I; in particular, frozen EcoP15I of the art is inactive after thawing out.

Thus, the technical problem underlying the present invention is to provide an efficient method and an expression system - e.g. a vector - for large scale production of storable type III restriction enzymes, particularly EcoP15I, whereby the product purified to homogeneity should be substantially the restriction endonuclease (Mod₂Res₂) and not the mixture of DNA methyltransferase (Mod₂) and restriction endonuclease (Mod₂Res₂).

This problem is solved by the provision of the embodiments as defined in the claims.

Briefly, the present invention solves this problem by providing:
- a recombinant DNA molecule comprising (i) a nucleotide sequence encoding a type III restriction enzyme, particularly EcoP15I, and (ii) a nucleotide sequence encoding an affinity-tag, preferably comprising histidine residues, whereby the affinity-tag is located on (a) Res-subunit of the enzyme and/or on (b) C terminus of the enzyme or subunits thereof,
- a vector comprising the recombinant DNA, in particular the vector: pQE-16, and
- a method of producing a type III restriction enzyme, particularly EcoP15I, comprising fermenting a microorganism transformed with said vector for the expression of type III restriction enzyme comprising at least another sequence encoding an affinity-tag, whereby the tag is added (i) to a C terminus of the enzyme or the subunits of the enzyme or (ii) to the Res-subunit of the enzyme in a medium under conditions suitable for the production of the enzyme, wherein the microorganism produces the enzyme and collecting the enzyme, which is produced.

It has been surprisingly discovered that a type III restriction enzyme, particularly EcoP15I, produced by the method of the invention is a more storable enzyme than well known enzymes, particularly EcoP15I. The enzyme of the invention can be stored with -20 degrees for over one year. Further, the enzyme purified by the method of the invention is particularly the restriction endonuclease (Mod₂Res₂) and not the mixture of DNA methyltransferase (Mod₂) and restriction endonuclease (Mod₂Res₂).

The present invention therefore relates to a storable type III restriction enzyme, particularly EcoP15I, and to the use of the enzyme of the invention (e.g. EcoP15I) as tool for biological analyses.

In addition, the method of the invention can be effectively used to generate high concentrated type III restriction enzymes e.g. EcoP15I, purified to homogeneity with an optimized shelf-life. Surprisingly, e.g. for 1 mg purified EcoP15I only 25 ml bacterial culture solution are necessary and the whole process of purification takes 2 days, whereby yield of the enzyme is 5 mg EcoP15I per 1 g cells.

Also claimed is a kit for biological analyses comprising the said enzyme e.g. EcoP15I, and/or synthetic analogues, modifications and active fragments thereof and an information about the using of parts of the kit.

Before the present compositions, formulations and methods are described, it is to be understood that this invention is not limited to the particular methods, compositions, and cell lines and vectors described herein, as such methods, compositions, and cell lines may, of course, vary. It is also to be understood that the terminology used herein is for the purpose of describing particular embodiments only, and is not intended to limit the scope of the present invention which is only defined by the appended claims.

As used herein, including the appended claims, singular forms of words such as "a," "an," and "the" include their corresponding plural referents unless the context clearly dictates otherwise. Thus, e.g., reference to "a vector" includes one or more different vectors, reference to "a host cell" includes one or more of such cells, and reference to "a method" includes reference to equivalent steps and methods known to a person of ordinary skill in the art, and so forth.

Unless otherwise defined, all technical and scientific terms used herein have the same meaning as commonly understood by a person of ordinary skill in the art to which this invention belongs. Although methods and materials similar or equivalent to those described herein can be used in the practice or testing of the present invention, suitable methods and materials are described below. All publications, patent applications, patents, and other references discussed above are provided solely for their disclosure prior to the filing date of the present application. Nothing herein is to be construed as an admission that the invention is not entitled to antedate such disclosure by virtue of its prior invention. All publications, patent applications, patents, and other references mentioned herein are incorporated by reference in their entirety including all figures and drawings.

As used herein with respect to nucleic acids, the term "isolated" means: (i) amplified in vitro by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art.

As used herein with respect to EcoP15I or EcoP15I-polypeptides (e.g. fragments) or EcoP15I/affinity-tag complex, the term "isolated" means separated from its native environment in sufficiently pure form so that it can be manipulated or used for any one of the purposes of the invention. Thus, isolated means sufficiently pure to be used (i) to raise and/or isolate antibodies, (ii) as a reagent in an assay, or (iii) for sequencing, etc.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., beta galactosidase or alkaline phosphatase), and genes which visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined. Such a vector in the context of the invention may be, e.g., a plasmid, cosmid, virus, phagemide, bacteriophage or another vector used e.g. conventionally in genetic engineering or in transfection of mammal cells and may comprise further genes such as marker genes which allow for the selection of said vector in a suitable host cell and under suitable conditions. Said vector may be one selected from commercially available vectors. Nonlimiting examples include plasmid vectors compatible with mammalian cells, such as pUC, pBluescript (Stratagene), pET (Novagen), pREP (Invitrogen), pCRTopo (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, pUCTag, pIZD35, pLXIN and pSIR (Clontech) and p1RES-EGFP (Clontech). Furthermore, the vectors may comprise expression control elements, allowing proper expression of the coding regions in suitable hosts. Such control elements are known to the artisan and may include a promoter, translation initiation codon, translation and insertion site or internal ribosomal entry sites (IRES) (Owens, Proc. Natl. Acad. Sci. USA 98 (2001), 1471-1476) for introducing an insert into the vector. Control elements ensuring expression in eukaryotic and prokaryotic cells are well known to those skilled in the art. As mentioned above, they usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression comprise for example the CMV-HSV thymidine kinase promoter, SV40, RSV-promoter (Rous sarcome virus), human elongation factor 1α-promoter, CMV enhancer, CaM-kinase promoter or SV40-enhancer. For the expression in the cells/cell lines of the present invention, several regulatory sequences are well known in the art. For the expression in prokaryotic cells, a multitude of promoters including, for example, the tac-lac-promoter, the lacUV5 or the trp promoter, has been described. Beside elements which are responsible for the initiation of transcription such regulatory elements may also comprise transcription termination signals, such as SV40-poly-A site or the tk-poly-A site, downstream of the polynucleotide. In this context, suitable expression vectors are known in the art such as Okayama-Berg cDNA expression vector pcDV1 (Pharmacia), pRc/CMV, pcDNA1, pcDNA3 (Invitrogene, as used, inter alia in the appended examples), pSPORT1 (GIBCO BRL) or pGEMHE (Promega), or prokaryotic expression vectors, such as lambda gt11. An expression vector according to this invention is at least capable of directing the replication, and preferably the expression, of nucleic acids contained therein. Suitable origins of replication include, for example, the ColE1, the SV40 viral and the M13 origins of replication. Suitable promoters include, for example, the cytomegalovirus (CMV) promoter, the lacZ promoter, the gal10 promoter and the Autographa californica multiple nuclear polyhedrosis virus (AcMNPV) promoter. Suitable termination sequences include, for example, the bovine growth hormone, SV40, lacZ and AcMNPV polyadenylation signals. Examples of selectable markers include neomycin, ampicillin, and hygromycin resistance and the like. Specifically-designed vectors allow the shuttling of DNA between different host cells, such as bacteria-animal cells. The vector may further comprise nucleic acid sequences encoding for secretion signals. Such sequences are well known to the person skilled in the art. Furthermore, depending on the expression system used, leader sequences capable of directing e.g. an expressed polypeptide to a cellular compartment may be added to the coding sequence of the nucleic acid molecules of the invention and are well known in the art. The leader sequence(s) is (are) assembled in appropriate phase with translation, initiation and termination sequences, and preferably, a leader sequence capable of directing secretion of translated protein, or a part thereof, into, inter alia, the extracellular membrane. Optionally, the heterologous sequence can encode a fusion protein including an C- or N-terminal identification peptide imparting desired characteristics, e.g., stabilization or simplified purification of expressed recombinant product. Once the vector has been incorporated into the appropriate host, the host is maintained under conditions suitable for high level expression of the nucleotide sequences, and, as desired, the collection and purification of the proteins, antigenic fragments or fusion proteins of the invention may follow. Of course, the vector can also comprise regulatory regions from pathogenic organisms.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Especially, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook and Russell, Molecular Cloning: A Laboratory Manual, Third Edition, Cold Spring Harbor Laboratory Press, 2001. Cells are genetically engineered by the introduction into the cells of heterologous DNA (RNA) encoding e.g. EcoP151 or EcoPI polypeptide or fragment or variant thereof. That heterologous DNA (RNA) is placed under operable control of transcriptional elements to permit the expression of the heterologous DNA in the host cell.

Preferred systems for mRNA expression in mammalian cells are those such as pRc/CMV (available from Invitrogen, Carlsbad, Calif.) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen, Carlsbad, Calif.), which contains an Epstein Barr virus (EBV) origin of replication, facilitating the maintenance of plasmid as a multicopy extrachromosomal element. Another expression vector is the pEF-BOS plasmid containing the promoter of polypeptide Elongation Factor 1 alpha, which stimulates efficiently transcription in vitro. The plasmid is described by Mishizuma and Nagata (Nuc. Acids Res. 18:5322, 1990), and its use in transfection experiments is disclosed by, for example, Demoulin (Mol. Cell. Biol. 16:4710-4716, 1996). Still another preferred expression vector is an adenovirus, described by Stratford-Perricaudet, which is defective for E1 and E3 proteins (J. Clin. Invest. 90:626-630, 1992). The use of the adenovirus as an Adeno.PlA recombinant is disclosed by Warnier et al., in intradermal injection in mice for immunization against P1A (Int. J. Cancer, 67:303-310, 1996).

The invention also embraces so-called expression kits, which allow the artisan to prepare a desired expression vector or vectors. Such expression kits include at least separate portions of each of the previously discussed coding sequences. Other components may be added, as desired, as long as the previously mentioned sequences, which are required, are included.

"An enzyme having biological activity" refers to enzymes exhibiting activity similar, but not necessarily identical to, an activity of EcoP15I or other type III restriction enzymes of invention, including mature forms, as measured in a particular biological assay, with or without dose dependency. In the case where dose dependency does exist, it need not be identical to that of the enzyme, but rather substantially similar to the dose-dependence in a given activity as compared to the EcoP15I of the present invention. Enzymes exhibiting similar activity refers to M.AfuORF1409P, BbrRORF912P, M.BbrRORF912P, BceSI, M.BceSI, Bc1229113ORF2P, Bc1229113ORF4P, Bc1229113ORF6P, M.Bc1229113ORF7P, BfaORFC143P, M.BfaORFC143P, BfaORFC196P, M.BfaORFC196P, BpeTORF3116P, M.BpeTORF3116P, BpsKORFC742P, M.BpsKORFC742P, M.BsupGET109P, CdpORF805P, M.CdpORF805P, CdpORF1895P, M.CdpORF1895P, M.Cje81116ORFEP, CteTORF908P, M.CteTORF908P, CteTORF1729P, M.CteTORF1729P, CthORFS4P, M.CthORFS4P, M.CthORFS206P, EcoCFTORF5372P, M.EcoCFTORF5372P, EcoPI, M.EcoPI, EcoP15I, M.EcoP15I, FacORFC158P, M.FacORFC158P, FnuKORF416P, M.FnuKORF416P, M.FnuVORFBP, FnuVORFCP, M.FnuVORFCP, GmeORFC20P, M.GmeORFC20P, HduORF1691P, M.HduORF1691P, HindORF1056P, M.HindORF1056P, HineI, HinfIII, M.HinfIII, M.Hpy99VII, Hpy1061P, M.Hpy1061P, M.Hpy787890P, M.Hpy788656P, M.Hpy788907P, Hpy789335P, Hpy789527P, M.Hpy790255P, Hpy790545P, Hpy790639P, M.HpyAX, HpyAXIP, M.HypAXI, HpyAORF1370P, M.HpyAORF1370P, HpyAORF1522P, M.HpyAORF1522P, Hpy99ORF1284P, M.Hpy99ORF1284P, Hpy99ORF1296P, M.Hpy99ORF1296P, Hpy99ORF1411P, M.Hpy99ORF1411P, M.Hpy166ORFHP, L1aFI, M.L1aFI, Mca43617ORFFP, M.Mca43617ORFFP, McaRIP, M.McaRIP, McaRIIP, M.McaRIIP, M.MmaGORF193P, M.MpeORF4770P, MpuCORF3960P, M.MpuCORF3960P, M.MpuCORF3970P, M.MpuCORF3980P, M.MpuCORF4800P, MspMCORFC186P, M.MspMCORFC186P, NeuORF2309P, M.NeuORF2309P, NgoAXP, M.NgoAXAP, M.NgoAXBP, NgoAORFC707P, M.NgoAORFC707P, NgoMX, M.NgoMX, NmeAORF1467P, M.NmeAORF1467P, NmeAORF1590P, M.NmeAORF1590P, NmeBORF1261P, M.NmeBORF1261P, NmeBORF1375P, M.NmeBORF1375P, NmeM1080P, PhaBI, M.PhaBI, PmaCCORF386P, M.PmaCCORF386P, PmuORF698P, M.PmuORF698P, PstII, M.PstII, RruMORFS3P, M.RruMORFS3P, StyCORF388P, M.StyCORF388P, StyLTI, M.StyLTI, StyLT20RF357P, M.StyLT2ORF357P, StyTORF872P, M.StyTORF872P, StyTORF2507P, M.StyTORF2507P, M.TdeORFC817P, TelBORF1481P, M.TelBORF1481P, TvoORF1464P, M.TvoORF1464P, TvoORF1476P, M.TvoORF1476P, M.XcaCORF214P, XcaCORF1068P, M.XcaCORF1068P, M.XfaORF1968P, M.XfaTORF833P and/or M.Yenp29930P.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37 degree C. in a solution comprising 6 times SSPE (20 times SSPE=3M NaCl; 0.2M NaH₂PO₄; 0.02M EDTA, pH 7.4), 0.5% SDS, 30% formamide, 100 ug/ml salmon sperm blocking DNA; followed by washes at 50 degree C. with 1.times.SSPE, 0.1 % SDS. In addition, to achieve even lower stringency, washes performed following stringent hybridization can be done at higher salt concentrations (e.g. 5 times SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

The enzyme of the present invention can be composed of amino acids joined to each other by peptide bonds or modified peptide bonds, i.e., peptide isosteres, and may contain amino acids other than the 20 gene-encoded amino acids. The enzymes may be modified by either natural processes, such as posttranslational processing, or by chemical modification techniques which are well known in the art. Such modifications are well described in basic texts and in more detailed monographs, as well as in a voluminous research literature. Modifications can occur anywhere in a enzyme, including the peptide backbone, the amino acid side-chains and the amino or carboxyl termini. It will be appreciated that the same type of modification may be present in the same or varying degrees at several sites in a given enzyme. Also, a given enzyme may contain many types of modifications. Enzymes may be branched, for example, as a result of ubiquitination, and they may be cyclic, with or without branching. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS--STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

The nucleic acid molecule according to the invention may be any type of nucleic acid, e.g. DNA or RNA. The DNA may, for example, be genomic DNA; synthetic DNA or cDNA. The RNA may be, e.g., mRNA. The nucleic acid molecule may be natural, synthetic or semisynthetic or it may be a derivative, such phosphorothioates. Furthermore, the nucleic acid molecule may be a recombinantly produced chimeric nucleic acid molecule comprising any of the aforementioned nucleic acid molecules either alone or in combination. The nucleic acid molecules comprised by said vector can be synthesized by standard methods, isolated from natural sources, or prepared as hybrids. Ligation of the coding sequences to transcriptional regulatory elements (e.g., promoters, enhancers, insulators or the like) and/or to other amino acid encoding sequences can be carried out using established methods. Said vector is introduced into the cells of the cell lines of the present invention by methods commonly known in the art, for example, lipofection, electroporation, Ca-Phosphate-transfection and the like. By a polynucleotide having a nucleotide sequence at least, for example, 70 %, 80 %, 85 %, preferably 90 %, more preferably 95 % "identical" to a reference nucleotide sequence encoding e.g. the EcoP15I of the present invention is intended that the nucleotide sequence of the polynucleotide is identical to the reference sequence except that the polynucleotide sequence may include up to five nucleotide mismatches per each 100 nucleotides of the reference nucleotide sequence encoding e.g. the EcoP15I. In other words, to obtain a polynucleotide having a nucleotide sequence at least 80 %, 85 %, 90 %, 95 % identical to a reference nucleotide sequence, up to 5 % of the nucleotides in the reference sequence may be deleted or substituted with another nucleotide, or a number of nucleotides up to 5 % of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

As indicated, nucleic acid molecules of the present invention which encode a type III restriction enzyme-affinity-tag complex may include, but are not limited to, those encoding the amino acid sequence of the polypeptide, by itself; the coding sequence for the polypeptide and additional sequences, such as those encoding an amino acid leader, such as a pre-, or pro-or prepro-protein sequence; the coding sequence of the polypeptide, with or without the aforementioned additional coding sequences, together with additional, non-coding sequences, including for example, but not limited to, introns and non-coding 5' and 3' sequences, such as the transcribed, non-translated sequences that play a role in transcription, mRNA processing, including splicing and polyadenylation signals, for example-ribosome binding and stability of mRNA; an additional coding sequence which codes for additional amino acids, such as those which provide additional functionalities. Thus, the sequence encoding the enzyme is fused to a marker sequence, such as a sequence encoding a peptide which facilitates purification of the fused polypeptide. In preferred embodiments of this aspect of the invention, the marker amino acid sequence is a hexa-histidine peptide, such as the tag provided in a pQE vector (Qiagen, Inc.), among others, many of which are commercially available. A preferred vector is pQE-16. Examples of further suitable expression vectors are pBTrp2, pBTac1 and pBTac2 (all available from Boehringer Mannheim), pKK233-2 (Pharmacia), pSE280 (Invitrogen), pGEMEX-1 (Promega), pQE-8 (QIAGEN), pQE-30 (QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK+ (Stratagene), pBluescript II SK-(Stratagene), pTrs30 (FERM BP-5407), pTrs32 (FERM BP-5408), pGHA2 (FERM BP-400), pGKA2 (FERM BP-6798), pTerm2 (Japanese Published Unexamined Patent Application No. 22979/91, U.S. Pat. No. 4,686,191, U.S. Pat. No. 4,939,094, U.S. Pat. No. 5,160,735), pEG400 [J. Bacteriol., 172, 2392 (1990)], pGEX (Pharmacia), pET system (Novagen), pSupex, pUB110, pTP5, pC194, pTrxFus (Invitrogen), pMAL-c2 (New England Biolabs), pUC18 [Gene, 33, 103 (1985)], pUC19 [Gene, 33, 103 (1985)], pSTV29 (Takara Shuzo Co., Ltd.), pSTV28 (Takara Shuzo Co., Ltd.), pUC118 (Takara Shuzo Co., Ltd.) and pPA1 (Japanese Published Unexamined Patent Application No. 233798/88). As the promoter, any promoters capable of functioning in host cells can be used. For example, promoters derived from Escherichia coli or phage, such as trp promoter (Ptrp), lac promoter (Plac), PL promoter, PR promoter and PSE promoter, SPO1 promoter, SPO2 promoter and penP promoter can be used. Artificially modified promoters such as a promoter in which two Ptrps are combined in tandem (Ptrp.times.2), tac promoter, letI promoter, lacT7 promoter and etc. can also be used. The transcription termination sequence is not essential for the expression of the desired DNA, but it is preferred that the transcription termination sequence lie immediately downstream of the structural gene.

As used herein, the term "hexa-histidine peptide" or "poly-histidine tract" or His-tag refers to the presence of two to ten histidine residues at either the amino- or carboxy-terminus of a nascent protein. As described in Gentz et al., Proc. Natl. Acad. Sci. USA 86:821-824 (1989), for instance, hexa-histidine provides for convenient purification of the fusion protein. The "HA" tag is another peptide useful for purification which corresponds to an epitope derived from the influenza hemagglutinin protein, which has been described by Wilson et al., Cell 37:767-778 (1984). As discussed below, other such fusion proteins include the EcoP15I fused to Fc at the N- or C terminus.

A poly-histidine tract of six to ten residues is most preferred. The poly-histidine tract is also defined functionally as being a number of consecutive histidine residues added to the protein of interest which allows the affinity purification of the resulting protein on a nickel-chelate column, or the identification of a protein terminus through the interaction with another molecule (e.g. an antibody reactive with the His-tag).

The DNA encoding type III restriction enzyme (e.g. EcoP15I) can be prepared from a microorganism belonging to the genus Escherichia. Examples of suitable microorganisms belonging to the genus Escherichia are Escherichia coli, Escherichia blattae, Escherichia fergusonii, Escherichia hermannii, Escherichia intermedia, Escherichia vulneris, etc. specifically, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110 (ATCC 27325), Escherichia coli NY49, Escherichia coli MP347, Escherichia coli NM522, Escherichia blattae ATCC 33430, Escherichia fergusonii ATCC 35473, Escherichia hermannii ATCC 33652, Escherichia intermedia ATCC 21073, Escherichia vulneris ATCC 39368, etc.

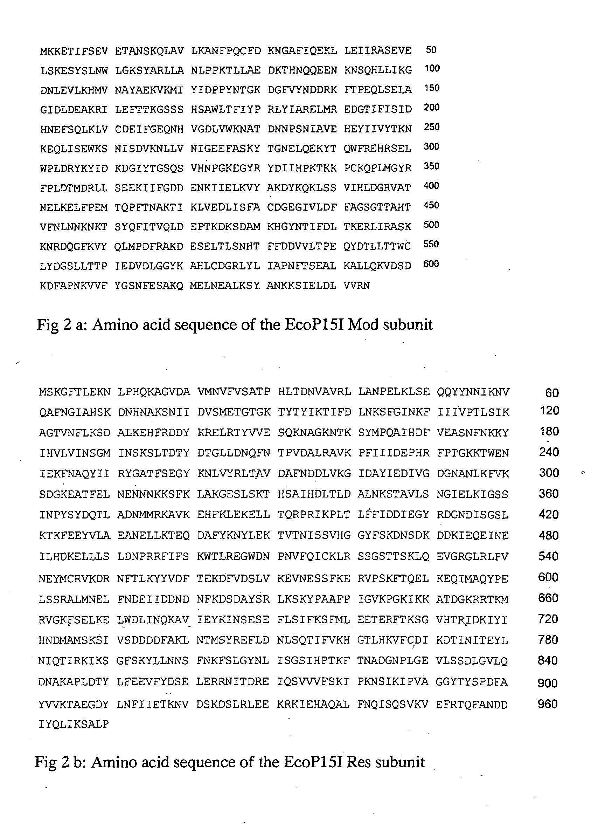

The present invention provides in one aspect a ready source of storable type III restriction enzyme, particularly EcoP15I. The invention relates also to a recombinant nucleic acid molecule (e.g. DNA molecule) comprising a first nucleotide sequence encoding the type III restriction enzyme (e.g. EcoP15I) and a second nucleotide sequence encoding an affinity-tag e.g. comprising histidine residues, whereby the tag is preferably added to Res-subunit of the enzyme (e.g. EcoP15I Fig. 2b).

In a preferred embodiment the histidine residues are 6xHis-tags. Further suitable nucleotide sequences encoding tags for purification and detection include beta-galactosidase-, protein A-, IgG-binding region of protein A-, chloramphenicol acetyltransferase-, poly(Arg)-, poly(Glu)-, protein G-, maltose-binding protein-, glutathione S-transferase-, S peptide-, DNA-binding protein domain-, Tac antigen-, thioredoxin-, green fluorescence protein-, and any antibody epitope-sequence [Akio Yamakawa, Jikken Igaku (Experimental Medicine), 13, 469-474 (1995)].

Type III restriction enzyme, particularly EcoP15I, variants of the invention may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. EcoP15I-polynucleotide variants can be produced for a variety of reasons, e.g., to optimize codon expression for a particular host (change codons in the human mRNA to those preferred by a bacterial host such as E. coli).

Naturally occurring EcoP15I or other type III restriction enzymes variants are called "allelic variants" and refer to one of several alternate forms of a gene occupying a given locus on a chromosome of an organism. These allelic variants can vary at either the polynucleotide and/or polypeptide level and are included in the present invention. Alternatively, non-naturally occurring variants may be produced by mutagenesis techniques or by direct synthesis.

EcoP15I is a preferred enzyme. Further enzymes in context of the invention embrace the following type III restriction endonucleases: M.AfuORF1409P, BbrRORF912P, M.BbrRORF912P, BceSI, M.BceSI, Bc1229113ORF2P, Bc1229113ORF4P, Bc1229113ORF6P, M.Bc1229113ORF7P, BfaORFC143P, M.BfaORFC143P, BfaORFC196P, M.BfaORFC196P, BpeTORF3116P, M.BpeTORF3116P, BpsKORFC742P, M.BpsKORFC742P, M.BsupGET109P, CdpORF805P, M.CdpORF805P, CdpORF1895P, M.CdpORF1895P, M.Cje81116ORFEP, CteTORF908P, M.CteTORF908P, CteTORF1729P, M.CteTORF1729P, CthORFS4P, M.CthORFS4P, M.CthORFS206P, EcoCFTORF5372P, M.EcoCFTORF5372P, EcoPI, M.EcoPI, EcoP15I, M.EcoP15I, FacORFC158P, M.FacORFC158P, FnuKORF416P, M.FnuKORF416P, M.FnuVORFBP, FnuVORFCP, M.FnuVORFCP, GmeORFC20P, M.GmeORFC20P, HduORF1691P, M.HduORF1691P, HindORF1056P, M.HindORF1056P, HineI, HinfIII, M.HinfIII, M.Hpy99VII, Hpy1061P, M.Hpy1061P, M.Hpy787890P, M.Hpy788656P, M.Hpy788907P, Hpy789335P, Hpy789527P, M.Hpy790255P, Hpy790545P, Hpy790639P, M.HpyAX, HpyAXIP, M.HypAXI, HpyAORF1370P, M.HpyAORF1370P, HpyAORF1522P, M.HpyAORF1522P, Hpy99ORF1284P, M.Hpy99ORF1284P, Hpy99ORF1296P, M.Hpy99ORF1296P, Hpy99ORF1411P, M.Hpy99ORF1411P, M.Hpy166ORFHP, L1aFI, M.L1aFI, Mca43617ORFFP, M.Mca43617ORFFP, McaRIP, M.McaRIP, McaRIIP, M.McaRIIP, M.MmaGORF193P, M.MpeORF4770P, MpuCORF3960P, M.MpuCORF3960P, M.MpuCORF3970P, M.MpuCORF3980P, M.MpuCORF4800P, MspMCORFC186P, M.MspMCORFC186P, NeuORF2309P, M.NeuORF2309P, NgoAXP, M.NgoAXAP, M.NgoAXBP, NgoAORFC707P, M.NgoAORFC707P, NgoMX, M.NgoMX, NmeAORF1467P, M.NmeAORF1467P, NmeAORF1590P, M.NmeAORF1590P, NmeBORF1261P, M.NmeBORF1261P, NmeBORF1375P, M.NmeBORF1375P, NmeM1080P, PhaBI, M.PhaBI, PmaCCORF386P, M.PmaCCORF386P, PmuORF698P, M.PmuORF698P, PstII, M.PstII, RruMORFS3P, M.RruMORFS3P, StyCORF388P, M.StyCORF388P, StyLTI, M.StyLTI, StyLT2ORF357P, M.StyLT2ORF357P, StyTORF872P, M.StyTORF872P, StyTORF2507P, M.StyTORF2507P, M.TdeORFC817P, TelBORF1481P, M.TelBORF1481P, TvoORF1464P, M.TvoORF1464P, TvoORF1476P, M.TvoORF1476P, M.XcaCORF214P, XcaCORF1068P, M.XcaCORF1068P, M.XfaORF1968P, M.XfaTORF833P and/or M.Yenp29930P.

The present invention relates also to a host cell comprising the vector of the invention. Examples of suitable host cells are cells of microorganisms belonging to the genera Escherichia, Serratia, Bacillus, Brevibacterium, Corynebacterium, Microbacterium, Pseudomonas, Agrobacterium, Alicyclobacillus, Anabaena, Anacystis, Arthrobacter, Azobacter, Chromatium, Erwinia, Methylobacterium, Phormidium, Rhodobacter, Rhodopseudomonas, Rhodospirillum, Scenedesmun, Streptomyces, Synnecoccus and Zymomonas. Preferred are microorganisms of the genera Escherichia, Bacillus, Brevibacterium, Corynebacterium, Pseudomonas, Agrobacterium, Alicyclobacillus, Anabaena, Anacystis, Arthrobacter, Azobacter, Chromatium, Erwinia, Methylobacterium, Phormidium, Rhodobacter, Rhodopseudomonas, Rhodospirillum, Scenedesmun, Streptomyces, Synnecoccus and Zymomonas.

As the host cell, any prokaryotic cells, yeast cells, animal cells, insect cells, plant cells, etc. that are capable of expressing the desired gene can be used. Animals and plants can also be used as hosts.

The expression vectors that can be employed are those capable of autonomous replication or integration into chromosome in the above host cells and comprising a promoter at a position appropriate for the transcription of the DNA e.g. encoding the EcoP15I or other type III restriction enzymes.

When a prokaryotic cell such as a bacterial cell is used as the host cell, it is preferred that the expression vector for expressing the above DNA is an expression vector which is capable of autonomous replication in the prokaryotic cell and which comprises a promoter, a ribosome binding sequence, the above DNA, and a transcription termination sequence. The vector may further comprise a gene regulating the promoter.

Specific examples of the above microorganisms are Escherichia coli XL1-Blue, Escherichia coli XL2-Blue, Escherichia coli DH1, Escherichia coli DH5.alpha., Escherichia coli MC1000, Escherichia coli KY3276, Escherichia coli W1485, Escherichia coli JM109, Escherichia coli HB101, Escherichia No. 49, Escherichia coli W3110, Escherichia coli NY49, Escherichia coli MP347, Escherichia coli NM522, Serratia ficaria, Serratia fonticola, Serratia liquefaciens, Serratia marcescens, Bacillus subtilis, Bacillus amyloliquefaciens, Corynebacterium ammoniagenes ATCC 6872, Brevibacterium immariophilum ATCC 14068, Brevibacterium saccharolyticum ATCC 14066, Brevibacterium flavum ATCC 14067, Brevibacterium lactofermentum ATCC 13869, Brevibacterium divaricatum ATCC 14020, Brevibacterium roseum ATCC 13825, Brevibacterium thiogenitalis ATCC 19240, Corynebacterium glutamicum ATCC 13032, Corynebacterium glutamicum ATCC 14297, Corynebacterium acetoacidophilum ATCC 13870, Corynebacterium acetoglutamicum ATCC 15806, Corynebacterium callunae ATCC 15991, Corynebacterium lilium ATCC 15990, Corynebacterium melassecola ATCC 17965, Microbacterium ammoniaphilum ATCC 15354, Pseudomonas sp. D-0110, Agrobacterium radiobacter, Agrobacterium rhizogenes, Agrobacterium rubi, Anabaena cylindrica, Anabaena doliolum, Anabaena flos-aquae, Arthrobacter aurescens, Arthrobacter citreus, Arthrobacter globformis, Arthrobacter hydrocarboglutamicus, Arthrobacter mysorens, Arthrobacter nicotianae, Arthrobacter paraffineus, Arthrobacter protophormiae, Arthrobacter roseoparaffinus, Arthrobacter sulfureus, Arthrobacter ureafaciens, Chromatium buderi, Chromatium tepidum, Chromatium vinosum, Chromatium warmingii, Chromatium fluviatile, Erwinia uredovora, Erwinia carotovora, Erwinia ananas, Erwinia herbicola, Erwinia punctata, Erwinia terreus, Methylobacterium rhodesianum, Methylobacterium extorquens, Phormidium sp. ATCC 29409, Rhodobacter capsulatus, Rhodobacter sphaeroides, Rhodopseudomonas blastica, Rhodopseudomonas marina, Rhodopseudomonas palustris, Rhodospirillum rubrum, Rhodospirillum salexigens, Rhodospirillum salinarum, Streptomyces ambofaciens, Streptomyces aureofaciens, Streptomyces aureus, Streptomyces fungicidicus, Streptomyces griseochromogenes, Streptomyces griseus, Streptomyces lividans, Streptomyces olivogriseus, Streptomyces rameus, Streptomyces tanashiensis, Streptomyces vinaceus and Zymomonas mobilis.

As a promotor, any promotor capable of functioning in prokaryotic cells can be used; e.g. T5 promotor/lac operator element.

Introduction of the recombinant DNA can be carried out by any of the methods for introducing DNA into the above host cells, for example, electroporation [Nucleic Acids Res., 16, 6127 (1988)], the method using calcium ion [Proc. Natl. Acad. Sci. USA, 69, 2110 (1972)], the protoplast method (Japanese Published Unexamined Patent Application No. 248394/88) and the methods described in Gene, 17, 107 (1982) and Molecular & General Genetics, 168, 111 (1979).

When a yeast cell is used as the host cell, YEp13 (ATCC 37115), YEp24 (ATCC 37051), YCp50 (ATCC 37419), pHS19, pHS15, etc. can be used as the expression vector.

As the promoter, any promoters capable of functioning in yeast cells can be used. Suitable promoters include PHO5 promoter, PGK promoter, GAP promoter, ADH promoter, gal 1 promoter, gal 10 promoter, heat shock protein promoter, MF.alpha.1 promoter, CUP1 promoter, etc.

Examples of suitable host cells are cells of yeast strains belonging to the genera Saccharomyces, Schizosaccharomyces, Kluyveromyces, Trichosporon, Schwanniomyces, Pichia, Candida, etc. specifically, Saccharomyces cerevisiae, Schizosaccharomyces pombe, Kluyveromyces lactis, Trichosporon pullulans, Schwanniomyces alluvius, Pichia pastoris and Candida utilis.

Introduction of the recombinant DNA can be carried out by any of the methods for introducing DNA into yeast cells, for example, electroporation [Methods in Enzymol., 194, 182 (1990)], the spheroplast method [Proc. Natl. Acad. Sci. USA, 81, 4889 (1984)], the lithium acetate method [J. Bacteriol., 153, 163 (1983)] and the method described in Proc. Natl. Acad. Sci. USA, 75, 1929 (1978).

When an animal cell is used as the host cell, pcDNAI/Amp, pcDNAI and pCDM8 (all available from Funakoshi), pAGE107 [Japanese Published Unexamined Patent Application No. 22979/91; Cytotechnology, 3, 133 (1990)], pREP4 (Invitrogen), pAGE103 [J. Biochem., 101, 1307 (1987)], pAGE210, pAMo, pAMoA [J. Biol. Chem., 268, 22782-22787 (1993), another name: pAMoPRSA (Japanese Published Unexamined Patent Application No. 336963/93)], pAS3-3 (Japanese Published Unexamined Patent Application No. 227075/90), etc. can be used as the expression vector.

As the promoter, any promoters capable of functioning in animal cells can be used. Suitable promoters include the promoter of IE (immediate early) gene of cytomegalovirus (human CMV), SV40 early promoter, the long terminal repeat promoter of moloney murine leukemia virus, the promoter of a retrovirus, heat shock promoter, SR.alpha. promoter, metallothionein promoter, etc. The enhancer of IE gene of human CMV may be used in combination with the promoter.

Examples of suitable host cells are mouse myeloma cells, rat myeloma cells, mouse hybridomas, Chinese hamster-derived CHO cells, BHK cells, African green monkey kidney cells, human-derived Namalwa cells and Namalwa KJM-1 cells, human embryonic kidney cells, human leukemia cells, HBT5637 (Japanese Published Unexamined Patent Application No. 299/88) and human large bowel cancer cell strains.

The mouse myeloma cells include SP2/0, NSO, etc.; the rat myeloma cells include YB2/0, etc.; the human embryonic kidney cells include HEK293 (ATCC: CRL-1573), etc.; the human leukemia cells include BALL-1, etc.; the African green monkey kidney cells include COS-1, COS-7, etc.; and the human large bowel cancer cell strains include HCT-15, etc.

Introduction of the recombinant DNA into animal cells can be carried out by any of the methods for introducing DNA into animal cells, for example, electroporation [Cytotechnology, 3, 133 (1990)], the calcium phosphate method (Japanese Published Unexamined Patent Application No. 227075/90), lipofection [Proc. Natl. Acad. Sci. USA, 84, 7413 (1987)], and the method described in Virology, 52, 456 (1973).

When an insect cell is used as the host cell, the protein can be expressed by using the methods described in Baculovirus Expression Vectors, A Laboratory Manual, W. H. Freeman and Company, New York (1992); Molecular Biology, A Laboratory Manual; Current Protocols in Molecular Biology, Supplement 1-38; Bio/Technology, 6, 47 (1988), etc.

That is, the recombinant gene transfer vector and a baculovirus are cotransfected into an insect cell to obtain a recombinant virus in the culture supernatant of the insect cell, and then an insect cell is infected with the recombinant virus, whereby the protein can be expressed.

Examples of the gene transfer vectors suitable for use in this method are pVL1392, pVL1393 and pBlueBacIII (products of Invitrogen).

An example of the baculovirus is Autographa californica nuclear polyhedrosis virus, which is a virus infecting insects belonging to the family Barathra.

Examples of the insect cells are ovarian cells of Spodoptera frugiperda, ovarian cells of Trichoplusia ni, and silkworm ovary-derived cell lines.

The ovarian cells of Spodoptera frugiperda include Sf9, Sf21 (Baculovirus Expression Vectors, A Laboratory Manual), etc; the ovarian cells of Trichoplusia ni include High 5, BTI-TN-5B1-4 (Invitrogen), etc; and the silkworm ovary-derived cell lines include Bombyx mori N4, etc.

Cotransfection of the above recombinant gene transfer vector and the above baculovirus into an insect cell for the preparation of the recombinant virus can be carried out by the calcium phosphate method (Japanese Published Unexamined Patent Application No. 227075/90), lipofection [Proc. Natl. Acad. Sci. USA, 84, 7413 (1987)], etc.

It is also possible to introduce the DNA into an insect cell by the same methods as used for introducing the DNA into an animal cell, for example, electroporation [Cytotechnology, 3, 133 (1990)], the calcium phosphate method (Japanese Published Unexamined Patent Application No. 227075/90) and lipofection [Proc. Natl. Acad. Sci. USA, 84, 7413 (1987)].

When a plant cell or plant is used as the host, the protein can be produced according to known methods [Soshiki Baiyo (Tissue Culture), 20 (1994); Soshiki Baiyo (Tissue Culture), 21 (1995); Trends in Biotechnology, 15, 45 (1997)].

Useful expression vectors include Ti plasmid, tobacco mosaic virus vector, etc. Culturing is usually carried out at pH 5-9 at 20-40 degree Celsius for 3-60 days. If necessary, antibiotics such as kanamycin and hygromycin may be added to the medium during the culturing.

It is also possible to produce the desired protein using an animal. For example, the desired protein can be produced in an animal carrying the introduced gene according to known methods [American Journal of Clinical Nutrition, 63, 639S (1996); American Journal of Clinical Nutrition, 63, 627S (1996); Bio/Technology, 9, 830 (1991)]. As the promoter, any promoters capable of functioning in an animal can be used. Preferred promoters include mammary gland cell-specific promoters such as alpha casein promoter, beta casein promoter, beta lactoglobulin promoter and whey acidic protein promoter. The desired protein can be produced by culturing the transformant derived from a microorganism, an animal cell or a plant cell carrying the recombinant vector comprising the DNA encoding the protein according to a conventional culturing method, allowing the protein to form and accumulate, and recovering the protein from the culture. When the transformant is an animal or plant, the protein can be produced by raising or culturing the animal or plant in a usual manner, allowing the protein to form and accumulate therein, and recovering the protein from the animal or plant main.

The present invention relates also to a peptide comprising the polynucleotide sequence encoding EcoP15I and an affinity-tag comprising histidine residues.

In a preferred embodiment of the invention, the affinity-tag is labeled with a molecule selected from the group consisting of radioactive molecule, fluorescent molecule, antibody, antibody fragment, hapten, carbohydrate, biotin, derivative of biotin, phosphorescent moiety, luminescent moiety, electrochemiluminescent moiety, chromatic moiety, and moiety having a detectable electron spin resonance, electrical capacitance, dielectric constant or electrical conductivity.

In another embodiment of the present invention the affinity-tag is located on C terminus of the enzyme or fragment (e.g. subunit) or polypeptide thereof; in a further embodiment of the invention the affinity-tag is located on the Res-subunit of the enzyme, preferably on the C terminus of the Res-subunit. The term located on is referred to: the tag (or the sequence encoding the tag) is added to, is close to or is attached to C terminus and/or the Res/(res)-subunit or the tag is in the proximity of the C terminus and/or the Res/(res)-subunit or the tag is a part of the C terminus and/or the Res/(res)-subunit.

Furthermore, the present invention relates to a Kit comprising the DNA of the invention, the vector, the host cell and/or the polypeptides and/or instructions for use or disposal of reagents in said kit.

Furthermore, the present invention relates also to a method for producing complete type III restriction enzyme, e.g. EcoP15I, characterized in that, (a) a cell is transformed with a sequence encoding EcoP15I or another type III restriction enzyme comprising at least another sequence encoding an affinity-tag, whereby the tag is added to the C terminus (e.g. of Res- or Mod-subunit; see Fig. 2a, b for subunits of EcoP15I) of the enzyme and/or (a') a cell is transformed with a sequence encoding EcoP15I or another type III restriction enzyme comprising at least another sequence encoding an affinity-tag, whereby the tag is added to the Res-subunit of the enzyme, and (b) the cells are cultured under conditions suitable for production of the enzyme e.g. EcoP15I, and (c) recovering the enzyme e.g. EcoP15I. That means the recombinant DNA is introduced into a host cell suited for the expression vector, whereby a transformant which produces the complete and pure or homogeneous protein having type III restriction enzyme-activity can be obtained.

In one embodiment of the method the affinity-tag is selected from the group comprising beta-galactosidase, protein A, IgG-binding region of protein A, chloramphenicol acetyltransferase, poly(Arg), poly(Glu), protein G, maltose-binding protein, glutathione S-transferase, polyhistidine chain, S peptide, DNA-binding protein domain, Tac antigen, thioredoxin, green fluorescence protein, and/or an antibody epitope comprising histidine residues. In a preferred embodiment the polyhistidine chain is a 6xHis-tag.

In another embodiment of the method of the invention the sequences encoding EcoP15I and the affinity-tag are incorporated in an over-expression vector.

In a further embodiment of the method the overexpression vector is a pQE-TriSystem vector, in particular pQE-16.

In another embodiment of the invention, the method for production of EcoP15I comprising the steps: (a) amplification of an operon encoding EcoP15I by PCR, whereby on 5' end a BamHI restriction site and on 3' end a BgIII - restriction site are incorporated, (b) incubation of product of (a) with BamHI and BgIII and isolation of a sequence encoding EcoP15I, (c) ligation of the sequence of (b) in pQE-16, (d) transformation of cells with the pQE-16 and expression of the EcoP15I, and (e) purification of the EcoP15I.

In a further embodiment, the purification according (e) comprising (i) isolation of the EcoP15I by nickel-nitrilo-tri-acetic acid affinity chromatography and (ii) heparin-sepharose chromatography.

Furthermore, the present invention relates to the use of said type III restriction enzyme (e.g. EcoP15I-affinity-tag complex) as tool for analysis of DNA, serial analysis of gene expression and/or the identification of corresponding genes.

In one embodiment of the present invention the said analysis is a determination of CAG repeats in the Huntington's disease genes.

In another embodiment of the present invention the gene expression profiling of two or more interacting cells, tissues, organs and/or organisms. In a preferred embodiment the organisms are hosts and pathogens.

The following examples are provided to describe the invention in further detail; these examples are intended to illustrate and not to limit the invention.

### Example

### Construction of the EcoP15I Overexpression Plasmid pQEP15

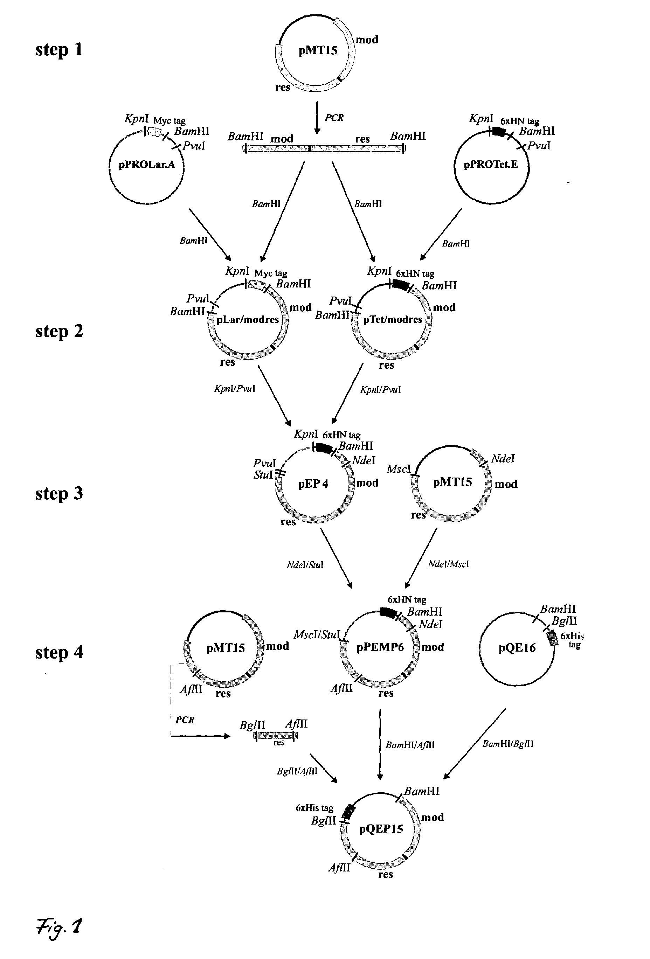

Starting from the plasmid pMT15 (Tierlich, 1995), the coding sequences of the EcoP15I *mod* and res genes were amplified by polymerase chain reaction using the forward primer 5'-ggcccggatccaaaaaagaaacgattttttc-3' and the reverse primer 5'-cgcgcggatcctgcgctcttgattaactgata-3' both introducing a recognition site for the restriction endonuclease BamHI (underlined) at the beginning of the mod gene and the end of the res gene, respectively. Purified PCR products were digested with BamHI and cloned into the BamHI-linearized expression vectors pPROLar.A and pPROTet.E (Clontech) respectively, resulting in the plasmids pLar/modres and pTet/modres, respectively (Fig. 1, step 1). The plasmid pPROLar.A provides a c-Myc epitop tag, the plasmid pPROTet.E a 6xHN affinity-tag for protein purification, both located upstream of the multicloning site. In addition, the modular nature of the expression vectors pPROLar.A and pPROTet.E provides the opportunity to design other vectors by substituting for individual modules.

To combine the promoter of pPROLar, which allows a particular tight control of gene expression, with the 6xHN-tag of pPROTet that enables EcoP15I purification under native conditions, plasmid pTet/modres was cleaved with the restriction endonucleases KpnI and PvuI to obtain the EcoP15I coding sequence with the 6xHN tag at its 5' end. This fragment was ligated with KpnI/PvuI-digested plasmid pLar/modres yielding the plasmid pEP4 (Fig. 1, step 2). Sequencing of the mod gene of pEP4 revealed a mutation near to its 3' end, which caused an amino acid substitution and possibly was responsible for the inactivity of the purified enzyme expressed by pEP4. To eliminate this mutation, a NdeI/MscI-fragment of pMT15 (Tierlich, 1995) containing the EcoP15I operon downstream of the NdeI site was ligated with the NdeI/StuI-cleaved plasmid pEP4 resulting in the plasmid pEMP6 (Fig. 1, step 3). Ligation was practicable, as MscI and StuI fragments are both blunt ended. Unfortunately, N-terminally 6xHN-tagged EcoP15I expressed from pEMP6 always showed less enzymatic activity compared to the untagged enzyme. Because the Mod subunit of type III restriction endonucleases mediates specific DNA recognition, we assumed that the N-terminal affinity-tag might interfere with the enzyme function.

Therefore, we removed the 6xHN-tag from the EcoP15I Mod subunit by proteolytic cleavage with enterokinase that recognizes the amino acid sequence DDDDK that does not exist in the EcoP15I amino acid sequence. The unique cleavage position for the protease is provided by the vector. Surprisingly, enterokinase cleavage nearly led to a loss of EcoP15I function. Examining the amino acid sequence of both, the Mod and Res subunit, turned out the sequence DDDDF at the C terminus of the Res subunit, which is similar to the cognate cleavage site. Analyzing proteolytic cleavage by SDS-PAGE, we found that in the presence of enterokinase the Res subunit (104 kDa) was digested into two fragments of about 33 KDa and about 80 kDa, which caused the reduction in enzymatic activity.
To circumvent the described difficulties, we re-cloned the EcoP15I operon into the BamHI/BglII.digested overexpression vector pQE-16 (Qiagen) that, in contrast to the above described constructs, provides a 6xHis affinity-tag to the C-term of the Res subunit of EcoP15I. To this aim, we had to introduce a BglII recognition sequence to the 3' terminus of the EcoP15I coding sequence via PCR using the forward primer 5'-caagagcttaaggaacaaatcatggctcagta-3' containing a recognition site for the restriction endonuclease AflII (underlined) and the reverse primer 5'-cgcgcagatcttggtaatgcgctcttgattaa-3' containing a recognition site for the restriction endonuclease BglII (underlined). The purified PCR-product was cleaved with the restriction endonucleases BglII and AflII. The 5' terminal 3800 bp of the EcoP15I coding sequence were obtained by digestion of the plasmid pEMP6 with BamHI and AflII. Both fragments were ligated with the BamHI and BglII cleaved expression vector pQE-16 in a one-step reaction yielding the plasmid pQEP15 (Fig. 2, step 4). To minimize the risk of mutations during a long range PCR, we merely amplified the 3' terminal EcoP15I 1100-bp sequence.

### EcoP15I expression and purification

LB media (80 ml) containing 100 µg/ml ampicillin was inoculated with 1 ml overnight culture of Escherichia coli strain JM109 (pQEP15). After growth for 3.5 h at 37 °C, expression of the EcoP15I Operon was induced with 1 mM IPTG and grown for additional 3.5 h at 30 °C. Cells were harvested by centrifugation at 3,500 x g for 20 min at 4 °C. EcoP15I was purified by two chromatographic steps: The cell pellet (0.6 g) was resuspended in 4 ml sonication buffer and 3 ml sonication buffer with 2 mg/ml lysozym as well as 0.2 µM PMSF were added. Cells were shaken in an ice bath for 30 min and disrupted by 4 x 15 sec sonication with 2 min-pauses. The lysate was centrifuged at 16,000xg at 4 °C for 45 min. The supernatant was taken and centrifuged again at 16,000xg at 4 °C for 20 min. The crude lysate (7.5 ml) was mixed with 1.5 ml Ni-NTA resin, which was pre-washed twice with 10 volumes sonication buffer, and incubated on ice for 30 min. During this time the mixture was gently shaken at times and then poured to a polypropylene column according to the instructions of the supplier (Qiagen). The column was successively washed twice with 10 ml sonication buffer, with 10 ml wash buffer and with 10 ml wash buffer including 10 mM imidazole. The bound EcoP15I restriction endonuclease was eluted from the Ni-NTA matrix with wash buffer containing 110 mM imidazole in 0.6 ml-fractions. Fractions with highest level of EcoP15I catalytic activity were pooled, diluted with 12 ml buffer H (20 mM Tris-HCl pH 7.4; 1 mM EDTA; 7mM β-mercaptoethanol) and loaded on a 1 ml Heparin Sepharose column (Amersham Biosciences), which was equilibrated previously with 7 column volumes buffer H. The sample was loaded in smaller quantities. The flow rate was adjusted to 0.3 ml/min. The column was washed again with 3 ml buffer H and the enzyme was eluted by a 0-1 M NaCl gradient in buffer H in 400-µl fractions. EcoP15I eluted at 600 mM NaCl. The enzyme was dialyzed against 1000-fold volume dialysis buffer (20 mM Tris-HCl pH 7.6, 200 mM NaCl, 1 mM EDTA, 7 mM β- mercaptoethanol, 50 % glycerol).

### Test for catalytic activity of EcoP15I

Enzymatic activity of the crude lysate or the collected fractions after Ni-NTA- and Heparin Sepharose chromatography was checked by incubation of 300 ng (= 0.51 pmol EcoP15 recognition sites) HindIII-linearized pUC19 DNA with different amounts of lysate and fractions, respectively, at 37 °C for 45 min. Restriction fragments were using a chemilmager (Biozym Diagnostik GmbH, Hess. Oldendorf) and software Phoretix 1-D (Phoretix International, Newcastle).

As will be apparent to those skilled in the art in which the invention is addressed, the present invention may be embodied in forms other than those specifically disclosed above without departing from the spirit or essential characteristics of the invention. The particular embodiments of the present invention, described above, are therefore to be considered in all respects as illustrative and not restrictive. The scope of the present invention is as setforth in the appended claims rather than being limited to the examples contained in the foregoing description.

## Claims

1. A recombinant DNA molecule comprising
(i) a first nucleotide sequence encoding a type III restriction enzyme and
(ii) a second nucleotide sequence encoding an affinity-tag, whereby the affinity-tag is located on Res-subunit and/or C terminus of the enzyme.

2. The DNA according to claim 1,
**characterized in that**,
- the enzyme is EcoP15I and/or
- the affinity-tag comprising histidine residues.

3. The DNA according to claim 1 or 2,
**characterized in that**,
the histidine residues are 6xHis-tags.

4. A vector comprising the recombinant molecule according to claims 1 - 3.

5. The vector according to claim 4,
**characterized in that**,
the vector is an over-expression vector, in particular pQE-16.

6. A host cell comprising the vector according to claim 4 or 5.

7. A polypeptide comprising (i) a sequence of a type III restriction enzyme, particularly EcoP15I, and (ii) an affinity-tag, whereby the affinity-tag is located on Res-subunit and/or C terminus of the enzyme.

8. The polypeptide according to claim 7,
**characterized in that**,
the affinity-tag comprising histidine residues.

9. A polypeptide according to claims 7 - 8,
**characterized in that**,
the affinity-tag is labeled with a molecule selected from the group consisting of radioactive molecule, fluorescent molecule, antibody, antibody fragment, hapten, carbohydrate, biotin, derivative of biotin, phosphorescent moiety, luminescent moiety, electrochemiluminescent moiety, chromatic moiety, and moiety having a detectable electron spin resonance, electrical capacitance, dielectric constant or electrical conductivity.

10. Kit comprising
a) a DNA according to anyone of claims 1 - 3, a vector of claim 4 or 5, a host cell of claim 6 and/or a polypeptide according to anyone of claims 7 - 9, and
b) instructions for use or disposal of reagents in said kit.

11. A method of producing a type III restriction enzyme, particularly EcoP15I, comprising fermenting a microorganism, in which at least one nucleotide sequence encoding the enzyme, which additionally comprises another sequence encoding an affinity-tag located on Res-subunit and/or C terminus of the enzyme is amplified, in a medium under conditions suitable for the production of the enzyme, wherein the microorganism produces the enzyme, and collecting the enzyme, which is produced.

12. The method for production of EcoP15I according to claim 11,
**characterized in that**,
the affinity-tag is selected from the group comprising beta-galactosidase, protein A, IgG-binding region of protein A, chloramphenicol acetyltransferase, poly(Arg), poly(Glu), protein G, maltose-binding protein, glutathione S-transferase, polyhistidine chain, S peptide, DNA-binding protein domain, Tac antigen, thioredoxin, green fluorescence protein, and/or any antibody epitope-sequence.

13. The method for production of EcoP15I according to claims 11 or 12,
**characterized in that**,
the polyhistidine chain is a 6xHis-tag.

14. The method for production of EcoP15I according to anyone of claims 11 - 13,
**characterized in that**,
the sequences encoding EcoP151 and the affinity-tag are incorporated in an over-expression vector.

15. The method for production of EcoP151 according to anyone of claims 11 - 14,
**characterized in that**,
the overexpression vector is a pQE-TriSystem vector, in particular pQE-16.

16. The method for production of EcoP15I according to anyone of claims 11 - 15 comprising
(a) amplification of a operon encoding EcoP15I by PCR, whereby on 5' end a BamHI restriction site and on 3' end a BgIII restriction site are incorporated,
(b) incubation of product of (a) with BamHI and BgIII and isolation of a sequence encoding EcoP15I,
(c) ligation of the sequence of (b) in pQE-16,
(d) transformation of cells with the pQE-16 and expression of the EcoP15I,
(e) purification of the EcoP15I.

17. The method according to claim 16,
**characterized in that**,
the purification according (e) comprising
(i) isolation of the EcoP15I by nickel-nitrilo-triacetic acid affinity chromatography and
(ii) heparin-sepharose chromatography.

18. Use of EcoP15I according to anyone of claims 7 - 9 as tool for analysis of DNA, serial analysis of gene expression and/or the identification of corresponding genes.

19. The use according to claim 18,
**characterized in that**,
the analysis is a determination of CAG repeats in the Huntington's disease genes.

20. The use according to claim 18 or 19,
**characterized in that**,
the gene expression profiling of two or more interacting cells, tissues, organs and/or organisms.

21. The use according to claim 20,
**characterized in that**,
the organisms are hosts and pathogens.


SEQUENCE LISTING

<110> Charite  Universitätsmedizin Berlin

<120> EcoP15I

<130> P251904EP-La

<140> P251904EP
<141> 2004-04-07

<160> 2

<170> PatentIn Ver. 2.1

<210> 1
<211> 644
<212> PRT
<213> Artificial Sequence

<220>
<223> Description of Artificial Sequence: EcoP15I Mod subunit

<400> 1
Met Lys Lys Glu Thr Ile Phe Ser Glu Val Glu Thr Ala Asn Ser Lys
  1               5                  10                  15

Gln Leu Ala Val Leu Lys Ala Asn Phe Pro Gln Cys Phe Asp Lys Asn
             20                  25                  30

Gly Ala Phe Ile Gln Glu Lys Leu Leu Glu Ile Ile Arg Ala Ser Glu
         35                  40                  45

Val Glu Leu Ser Lys Glu Ser Tyr Ser Leu Asn Trp Leu Gly Lys Ser
     50                  55                  60

Tyr Ala Arg Leu Leu Ala Asn Leu Pro Pro Lys Thr Leu Leu Ala Glu
 65                  70                  75                  80

Asp Lys Thr His Asn Gln Gln Glu Glu Asn Lys Asn Ser Gln His Leu
                 85                  90                  95

Leu Ile Lys Gly Asp Asn Leu Glu Val Leu Lys His Met Val Asn Ala
            100                 105                 110

Tyr Ala Glu Lys Val Lys Met Ile Tyr Ile Asp Pro Pro Tyr Asn Thr
        115                 120                 125

Gly Lys Asp Gly Phe Val Tyr Asn Asp Asp Arg Lys Phe Thr Pro Glu
    130                 135                 140

Gln Leu Ser Glu Leu Ala Gly Ile Asp Leu Asp Glu Ala Lys Arg Ile
145                 150                 155                 160

Leu Glu Phe Thr Thr Lys Gly Ser Ser Ser His Ser Ala Trp Leu Thr
                165                 170                 175

Phe Ile Tyr Pro Arg Leu Tyr Ile Ala Arg Glu Leu Met Arg Glu Asp
            180                 185                 190

Gly Thr Ile Phe Ile Ser Ile Asp His Asn Glu Phe Ser Gln Leu Lys
        195                 200                 205

Leu Val Cys Asp Glu Ile Phe Gly Glu Gln Asn His Val Gly Asp Leu
    210                 215                 220

Val Trp Lys Asn Ala Thr Asp Asn Asn Pro Ser Asn Ile Ala Val Glu
225                 230                 235                 240

His Glu Tyr Ile Ile Val Tyr Thr Lys Asn Lys Glu Gln Leu Ile Ser
                245                 250                 255

Glu Trp Lys Ser Asn Ile Ser Asp Val Lys Asn Leu Leu Val Asn Ile
            260                 265                 270

Gly Glu Glu Phe Ala Ser Lys Tyr Thr Gly Asn Glu Leu Gln Glu Lys
        275                 280                 285

Tyr Thr Gln Trp Phe Arg Glu His Arg Ser Glu Leu Trp Pro Leu Asp
    290                 295                 300

Arg Tyr Lys Tyr Ile Asp Lys Asp Gly Ile Tyr Thr Gly Ser Gln Ser
305                 310                 315                 320

Val His Asn Pro Gly Lys Glu Gly Tyr Arg Tyr Asp Ile Ile His Pro
                325                 330                 335

Lys Thr Lys Lys Pro Cys Lys Gln Pro Leu Met Gly Tyr Arg Phe Pro
            340                 345                 350

Leu Asp Thr Met Asp Arg Leu Leu Ser Glu Glu Lys Ile Ile Phe Gly
        355                 360                 365

Asp Asp Glu Asn Lys Ile Ile Glu Leu Lys Val Tyr Ala Lys Asp Tyr
    370                 375                 380

Lys Gln Lys Leu Ser Ser Val Ile His Leu Asp Gly Arg Val Ala Thr
385                 390                 395                 400

Asn Glu Leu Lys Glu Leu Phe Pro Glu Met Thr Gln Pro Phe Thr Asn
                405                 410                 415

Ala Lys Thr Ile Lys Leu Val Glu Asp Leu Ile Ser Phe Ala Cys Asp
            420                 425                 430

Gly Glu Gly Ile Val Leu Asp Phe Phe Ala Gly Ser Gly Thr Thr Ala
        435                 440                 445

His Thr Val Phe Asn Leu Asn Asn Lys Asn Lys Thr Ser Tyr Gln Phe
    450                 455                 460

Ile Thr Val Gln Leu Asp Glu Pro Thr Lys Asp Lys Ser Asp Ala Met
465                 470                 475                 480

Lys His Gly Tyr Asn Thr Ile Phe Asp Leu Thr Lys Glu Arg Leu Ile
                485                 490                 495

Arg Ala Ser Lys Lys Asn Arg Asp Gln Gly Phe Lys Val Tyr Gln Leu
            500                 505                 510

Met Pro Asp Phe Arg Ala Lys Asp Glu Ser Glu Leu Thr Leu Ser Asn
        515                 520                 525

His Thr Phe Phe Asp Asp Val Val Leu Thr Pro Glu Gln Tyr Asp Thr
    530                 535                 540

Leu Leu Thr Thr Trp Cys Leu Tyr Asp Gly Ser Leu Leu Thr Thr Pro
545                 550                 555                 560

Ile Glu Asp Val Asp Leu Gly Gly Tyr Lys Ala His Leu Cys Asp Gly
                565                 570                 575

Arg Leu Tyr Leu Ile Ala Pro Asn Phe Thr Ser Glu Ala Leu Lys Ala
            580                 585                 590

Leu Leu Gln Lys Val Asp Ser Asp Lys Asp Phe Ala Pro Asn Lys Val
        595                 600                 605

Val Phe Tyr Gly Ser Asn Phe Glu Ser Ala Lys Gln Met Glu Leu Asn
    610                 615                 620

Glu Ala Leu Lys Ser Tyr Ala Asn Lys Lys Ser Ile Glu Leu Asp Leu
625                 630                 635                 640

Val Val Arg Asn

<210> 2
<211> 970
<212> PRT
<213> Artificial Sequence

<220>
<223> Description of Artificial Sequence: EcoP15I Res subunits

<400> 2
Met Ser Lys Gly Phe Thr Leu Glu Lys Asn Leu Pro His Gln Lys Ala
  1               5                  10                  15

Gly Val Asp Ala Val Met Asn Val Phe Val Ser Ala Thr Pro His Leu
            20                  25                  30

Thr Asp Asn Val Ala Val Arg Leu Leu Ala Asn Pro Glu Leu Lys Leu
        35                  40                  45

Ser Glu Gln Gln Tyr Tyr Asn Asn Ile Lys Asn Val Gln Ala Phe Asn
    50                  55                  60

Gly Ile Ala His Ser Lys Asp Asn His Asn Ala Lys Ser Asn Ile Ile
65                  70                  75                  80

Asp Val Ser Met Glu Thr Gly Thr Gly Lys Thr Tyr Thr Tyr Ile Lys

                85                  90                  95

Thr Ile Phe Asp Leu Asn Lys Ser Phe Gly Ile Asn Lys Phe Ile Ile
            100                 105                 110

Ile Val Pro Thr Leu Ser Ile Lys Ala Gly Thr Val Asn Phe Leu Lys
        115                 120                 125

Ser Asp Ala Leu Lys Glu His Phe Arg Asp Asp Tyr Lys Arg Glu Leu
    130                 135                 140

Arg Thr Tyr Val Val Glu Ser Gln Lys Asn Ala Gly Lys Asn Thr Lys
145                 150                 155                 160

Ser Tyr Met Pro Gln Ala Ile His Asp Phe Val Glu Ala Ser Asn Phe
                165                 170                 175

Asn Lys Lys Tyr Ile His Val Leu Val Ile Asn Ser Gly Met Ile Asn
            180                 185                 190

Ser Lys Ser Leu Thr Asp Thr Tyr Asp Thr Gly Leu Leu Asp Asn Gln
        195                 200                 205

Phe Asn Thr Pro Val Asp Ala Leu Arg Ala Val Lys Pro Phe Ile Ile
    210                 215                 220

Ile Asp Glu Pro His Arg Phe Pro Thr Gly Lys Lys Thr Trp Glu Asn
225                 230                 235                 240

Ile Glu Lys Phe Asn Ala Gln Tyr Ile Ile Arg Tyr Gly Ala Thr Phe
                245                 250                 255

Ser Glu Gly Tyr Lys Asn Leu Val Tyr Arg Leu Thr Ala Val Asp Ala
            260                 265                 270

Phe Asn Asp Asp Leu Val Lys Gly Ile Asp Ala Tyr Ile Glu Asp Ile
        275                 280                 285

Val Gly Asp Gly Asn Ala Asn Leu Lys Phe Val Lys Ser Asp Gly Lys
    290                 295                 300

Glu Ala Thr Phe Glu Leu Asn Glu Asn Asn Asn Lys Lys Ser Phe Lys
305                 310                 315                 320

Leu Ala Lys Gly Glu Ser Leu Ser Lys Thr His Ser Ala Ile His Asp
                325                 330                 335

Leu Thr Leu Asp Ala Leu Asn Lys Ser Thr Ala Val Leu Ser Asn Gly
            340                 345                 350

Ile Glu Leu Lys Ile Gly Ser Ser Ile Asn Pro Tyr Ser Tyr Asp Gln
        355                 360                 365

Thr Leu Ala Asp Asn Met Met Arg Lys Ala Val Lys Glu His Phe Lys
    370                 375                 380

Leu Glu Lys Glu Leu Leu Thr Gln Arg Pro Arg Ile Lys Pro Leu Thr
385                 390                 395                 400

Leu Phe Phe Ile Asp Asp Ile Glu Gly Tyr Arg Asp Gly Asn Asp Ile
                405                 410                 415

Ser Gly Ser Leu Lys Thr Lys Phe Glu Glu Tyr Val Leu Ala Glu Ala
            420                 425                 430

Asn Glu Leu Leu Lys Thr Glu Gln Asp Ala Phe Tyr Lys Asn Tyr Leu
        435                 440                 445

Glu Lys Thr Val Thr Asn Ile Ser Ser Val His Gly Gly Tyr Phe Ser
    450                 455                 460

Lys Asp Asn Ser Asp Lys Asp Asp Lys Ile Glu Gln Glu Ile Asn Glu
465                 470                 475                 480

Ile Leu His Asp Lys Glu Leu Leu Leu Ser Leu Asp Asn Pro Arg Arg
                485                 490                 495

Phe Ile Phe Ser Lys Trp Thr Leu Arg Glu Gly Trp Asp Asn Pro Asn
            500                 505                 510

Val Phe Gln Ile Cys Lys Leu Arg Ser Ser Gly Ser Thr Thr Ser Lys
        515                 520                 525

Leu Gln Glu Val Gly Arg Gly Leu Arg Leu Pro Val Asn Glu Tyr Met
    530                 535                 540

Cys Arg Val Lys Asp Arg Asn Phe Thr Leu Lys Tyr Tyr Val Asp Phe
545                 550                 555                 560

Thr Glu Lys Asp Phe Val Asp Ser Leu Val Lys Glu Val Asn Glu Ser
                565                 570                 575

Ser Phe Lys Glu Arg Val Pro Ser Lys Phe Thr Gln Glu Leu Lys Glu
            580                 585                 590

Gln Ile Met Ala Gln Tyr Pro Glu Leu Ser Ser Arg Ala Leu Met Asn
        595                 600                 605

Glu Leu Phe Asn Asp Glu Ile Ile Asp Asp Asn Asp Asn Phe Lys Asp
    610                 615                 620

Ser Asp Ala Tyr Ser Arg Leu Lys Ser Lys Tyr Pro Ala Ala Phe Pro
625                 630                 635                 640

Ile Gly Val Lys Pro Gly Lys Ile Lys Lys Ala Thr Asp Gly Lys Arg
                645                 650                 655

Arg Thr Lys Met Arg Val Gly Lys Phe Ser Glu Leu Lys Glu Leu Trp
            660                 665                 670

Asp Leu Ile Asn Gln Lys Ala Val Ile Glu Tyr Lys Ile Asn Ser Glu
        675                 680                 685

Ser Glu Phe Leu Ser Ile Phe Lys Ser Phe Met Leu Glu Glu Thr Glu
    690                 695                 700

Arg Phe Thr Lys Ser Gly Val His Thr Arg Ile Asp Lys Ile Tyr Ile
705                 710                 715                 720

His Asn Asp Met Ala Met Ser Lys Ser Ile Val Ser Asp Asp Asp Asp
                725                 730                 735

Phe Ala Lys Leu Asn Thr Met Ser Tyr Arg Glu Phe Leu Asp Asn Leu
            740                 745                 750

Ser Gln Thr Ile Phe Val Lys His Gly Thr Leu His Lys Val Phe Cys
        755                 760                 765

Asp Ile Lys Asp Thr Ile Asn Ile Thr Glu Tyr Leu Asn Ile Gln Thr
    770                 775                 780

Ile Arg Lys Ile Lys Ser Gly Phe Ser Lys Tyr Leu Leu Asn Asn Ser
785                 790                 795                 800

Phe Asn Lys Phe Ser Leu Gly Tyr Asn Leu Ile Ser Gly Ser Ile His
                805                 810                 815

Pro Thr Lys Phe Thr Asn Ala Asp Gly Asn Pro Leu Gly Glu Val Leu
            820                 825                 830

Ser Ser Asp Leu Gly Val Leu Gln Asp Asn Ala Lys Ala Pro Leu Asp
        835                 840                 845

Thr Tyr Leu Phe Glu Glu Val Phe Tyr Asp Ser Glu Leu Glu Arg Arg
    850                 855                 860

Asn Ile Thr Asp Arg Glu Ile Gln Ser Val Val Val Phe Ser Lys Ile
865                 870                 875                 880

Pro Lys Asn Ser Ile Lys Ile Pro Val Ala Gly Gly Tyr Thr Tyr Ser
                885                 890                 895

Pro Asp Phe Ala Tyr Val Val Lys Thr Ala Glu Gly Asp Tyr Leu Asn
            900                 905                 910

Phe Ile Ile Glu Thr Lys Asn Val Asp Ser Lys Asp Ser Leu Arg Leu
        915                 920                 925

Glu Glu Lys Arg Lys Ile Glu His Ala Gln Ala Leu Phe Asn Gln Ile
    930                 935                 940

Ser Gln Ser Val Lys Val Glu Phe Arg Thr Gln Phe Ala Asn Asp Asp
945                 950                 955                 960

Ile Tyr Gln Leu Ile Lys Ser Ala Leu Pro
                965                 970